# EUROPEAN PATENT APPLICATION

(11) **EP 4 640 171 A1**
(43) Date of publication of application: **29.10.2025**
(21) Application number: 23905623.7
(22) Date of filing: 27.11.2023
(51) Int. Cl.: A61B 17/42, A61B 90/00, A61B 17/3209, A61B 17/32, A61M 1/00

(54) **SURGICAL SYSTEM HAVING CAVITY DISTENTION, SHAVING AND ASPIRATION FUNCTIONS AND CONTROL METHOD**

(30) Priority: 22.12.2022 CN 202211658504
(71) Applicant: Scivita Medical Technology Co., Ltd., Suzhou, Jiangsu 215127 (CN)
(72) Inventor: CHIN, To, Suzhou, Jiangsu 215127 (CN)
(74) Representative: Nex & Phister Law & IP Aps
(86) International application number: PCT/CN2023/134458
(87) International publication number: WO 2024/131455

(57) **Abstract**

The present disclosure provides a surgical system having distension, shaving and suction functions comprise a controller, a distension device delivering distension fluid to a target cavity and performing a distension operation based on control signals of the controller, a shaving device performing a shaving operation in the target cavity based on the control signals of the controller, wherein the shaving device has a suction channel, and a collection device. The collection device comprises a distension fluid collection device collecting the distension fluid naturally discharged from the target cavity; and a negative pressure supply device supplying a negative pressure to the suction channel based on the control signals of the controller, such that the suction channel suctions tissue waste and fluid waste generated during the shaving operation. The present disclosure also provides a control method and a surgical apparatus having distension, shaving and suction functions.

## Description

### FIELD

The present disclosure relates to medical devices, and specifically relates to a surgical system having distension, shaving, and suction functions, a control method thereof, and a surgical device.

### BACKGROUND

Distension fluid is infused into a uterine cavity to expand the uterine cavity and an operational and visual space for the procedure is formed, before performing hysteroscopic surgery. During the surgery, intrauterine tumors and polyp tissues are resected using a shaving handle, while removes fluid waste and tissue waste are removed from the body by a negative pressure suction function.

The current equipment used for the hysteroscopic surgery mostly consists of a shaving device host, a distension device and a suction device (or a central negative pressure suction system), to accomplish the various functions required for the hysteroscopic surgery. The distension device is a separate component, and the distension device and the suction device are combined together.

The existing integrated machines for shaving, distension and suction merely combine these three functions without coordinated interaction among them, requiring the surgeon to manually control each function during the procedure.

### SUMMARY

The disclosure provides a surgical system having distension, shaving, and suction functions, a control method thereof, and a surgical device.

A first aspect of the present disclosure provides a surgical system having distension, shaving, and suction functions, comprising: a controller; a distension device configured to deliver distension fluid to a target cavity and perform a distension operation based on control signals of the controller; a shaving device configured to perform a shaving operation in the target cavity based on the control signals of the controller, wherein the shaving device has a suction channel; and a collection device, wherein the collection device comprises: a distension fluid collection device configured to collect the distension fluid naturally discharged from the target cavity; a negative pressure supply device configured to supply a negative pressure to the suction channel based on the control signals of the controller, such that the suction channel suctions tissue waste and fluid waste generated during the shaving operation; and a storage container configured to be in fluid communication with both the suction channel and the distension fluid collection device, and contain the tissue waste and the fluid waste from the suction channel and the distension fluid from the distension fluid collection device.

In the surgical system of at least one embodiment of the present disclosure, the distension device comprises: a distension fluid supply device configured to store the distension fluid; a first pumping device; and a first driving device configured to drive the first pumping device based on the control signals of the controller, such that the first pumping device delivers the distension fluid from the distension fluid supply device to the target cavity via a delivery pipe.

In the surgical system of at least one embodiment of the present disclosure, the distension device further comprises a pressure measurement device that measures a liquid pressure in the delivery pipe to monitor a liquid pressure in the target cavity.

In the surgical system of at least one embodiment of the present disclosure, the pressure measurement device comprises: a pressure chamber having a first wall and a second wall, wherein at least a portion of the delivery pipe is arranged between the first wall and the second wall; and at least one pressure sensor arranged on the first wall, the second wall or both the first wall and the second wall to measure the liquid pressure in the delivery pipe.

In the surgical system of at least one embodiment of the present disclosure, the delivery pipe is a flexible pipe.

In the surgical system of at least one embodiment of the present disclosure, the pressure sensor is arranged in an embedded manner on the first wall, the second wall or both the first wall and the second wall.

In the surgical system of at least one embodiment of the present disclosure, the liquid pressure measured by the pressure measurement device is transmitted to the controller in real time.

In the surgical system of at least one embodiment of the present disclosure, the first pumping device is equipped with a first microswitch, wherein the first microswitch generates a first trigger signal when the delivery pipe is connected with the first pumping device, and the first trigger signal is transmitted to the controller.

In the surgical system of at least one embodiment of the present disclosure, the first pumping device is a peristaltic pump, and the first driving device is a stepper motor.

In the surgical system of at least one embodiment of the present disclosure, the pressure chamber is equipped with a second microswitch, wherein the second microswitch generates a second trigger signal when the delivery pipe is properly arranged between the first wall and the second wall, and the second trigger signal is transmitted to the controller.

In the surgical system of at least one embodiment of the present disclosure, the shaving device comprises: shaving cutters having an outer cutter and an inner cutter, wherein the inner cutter is disposed within the outer cutter and coaxially aligned with the outer cutter; and a shaving operation device configured to drive the inner cutter to rotate relative to the outer cutter to perform the shaving operation, wherein the suction channel is formed within the inner cutter and the shaving operation device, and communicates the target cavity and the negative pressure supply device.

In the surgical system of at least one embodiment of the present disclosure, the inner cutter and the outer cutter are tubular, the inner cutter has a first end having a side opening serving as a blade of the inner cutter, the inner cutter has a second end being driven by the shaving operation device, the outer cutter has a first end having a side opening serving as a blade of the outer cutter, and the outer cutter has a second end being fixedly connected to the shaving operation device, wherein the side opening of the first end of the inner cutter and the side opening of the first end of the outer cutter have a same or similar axial position.

In the surgical system of at least one embodiment of the present disclosure, the storage container has a first port being in communication with the suction channel, the storage container has a second port being in communication with the negative pressure supply device, and the negative pressure supply device supplies the negative pressure to the suction channel via a gas passage arranged between the first port and the second port to suction the tissue waste and the fluid waste.

In the surgical system of at least one embodiment of the present disclosure, the distension fluid collection device is in communication with the first port of the storage container, and the negative pressure supply device supplies the negative pressure to the distension fluid collection device via the gas passage to collect the distension fluid naturally discharged from the target cavity.

In the surgical system of at least one embodiment of the present disclosure, the collection device further comprises a liquid metering device, and the liquid metering device measures a liquid volume in the storage container in real time and transmits the liquid volume to the controller in real time.

In the surgical system of at least one embodiment of the present disclosure, the shaving device further comprises an activation switch, the activation switch, based on an operation action, generates an activation signal for the shaving device and transmits the activation signal to the controller, and the controller, at least based on the activation signal of the shaving device, generates the motor drive signals to drive the motor 304 of the shaving operation device.

In the surgical system of at least one embodiment of the present disclosure, the activation switch is a foot-operated switch.

In the surgical system of at least one embodiment of the present disclosure, the distension device further comprises a flow detection device, and the flow detection device measures a volume of the distension fluid delivered to the target cavity by the distension device in real time and transmits the measurement value to the controller in real time.

In the surgical system of at least one embodiment of the present disclosure, the controller generates the control signals to control the first driving device, based at least on the first trigger signal of the first microswitch and the second trigger signal of the second microswitch.

In the surgical system of at least one embodiment of the present disclosure, the surgical system further comprises a first operation receiving device that receives user's operation actions to generate a first operation signal and transmits the first operation signal to the controller, and the controller generates the control signals to control the first driving device based on the first trigger signal, the second trigger signal and the first operation signal.

In the surgical system of at least one embodiment of the present disclosure, the surgical system further comprises a second operation receiving device that receives user' s operation actions to generate a second operation signal and transmits the second operation signal to the controller, and the controller generates the control signals to control the negative pressure supply device to supply the negative pressure based at least on the second operation signal.

In the surgical system of at least one embodiment of the present disclosure, the controller, at least based on the second operation signal, generates the control signals for controlling the negative pressure supply device to provide the negative pressure.

In the surgical system of at least one embodiment of the present disclosure, the surgical system further comprises a third operation receiving device that receives user' s operation actions to generate a third operation signal and transmits the third operation signal to the controller, the controller associates a preset operating mode for the shaving cutters based on the third operation signal, and the controller outputs a shaving control signal associated with the preset operating mode to control the shaving operation device to drive the inner cutter based on the third operation signal and an activation signal for the shaving device.

In the surgical system of at least one embodiment of the present disclosure, the shaving cutters enter the target cavity via an endoscope located outside the surgical system, and perform the shaving operation.

A second aspect of the present disclosure provides a surgical apparatus having distension, shaving, and suction functions, comprising: a controller; a distension device configured to deliver distension fluid to a target cavity to perform a distension operation based on control signals of the controller; a shaving operation device configured to drive a shaving device and perform a shaving operation in the target cavity based on the control signals of the controller, wherein the shaving device locates outside the surgical apparatus and has a suction channel; and a negative pressure supply device configured to supply a negative pressure based on the control signals of the controller, such that the suction channel of the shaving device suctions tissue waste and fluid waste generated during the shaving operation.

In the surgical apparatus of at least one embodiment of the present disclosure, the distension device comprises: a first pumping device; and a first driving device configured to drive the first pumping device based on the control signals of the controller, such that the first pumping device delivers the distension fluid from the distension fluid supply device to the target cavity via a delivery pipe.

In the surgical apparatus of at least one embodiment of the present disclosure, the distension device further comprises a pressure measurement device that measures a liquid pressure in the delivery pipe to monitor a liquid pressure in the target cavity.

In the surgical apparatus of at least one embodiment of the present disclosure, the pressure measurement device comprises: a pressure chamber having a first wall and a second wall, wherein at least a portion of the delivery pipe is arranged between the first wall and the second wall; and at least one pressure sensor arranged on the first wall, the second wall or both the first wall and the second wall to measure the liquid pressure in the delivery pipe.

In the surgical apparatus of at least one embodiment of the present disclosure, the delivery pipe is a flexible pipe.

In the surgical apparatus of at least one embodiment of the present disclosure, the pressure sensor is arranged in an embedded manner on the first wall, the second wall or both the first wall and the second wall.

In the surgical apparatus of at least one embodiment of the present disclosure, the liquid pressure measured by the pressure measurement device is transmitted to the controller in real time.

In the surgical apparatus of at least one embodiment of the present disclosure, the first pumping device is equipped with a first microswitch, wherein the first microswitch generates a first trigger signal when the delivery pipe is connected with the first pumping device, and the first trigger signal is transmitted to the controller.

In the surgical apparatus of at least one embodiment of the present disclosure, the first pumping device is a peristaltic pump, and the first driving device is a stepper motor.

In the surgical apparatus of at least one embodiment of the present disclosure, the pressure chamber is equipped with a second microswitch, wherein the second microswitch generates a second trigger signal when the delivery pipe is properly arranged between the first wall and the second wall, and the second trigger signal is transmitted to the controller.

A third aspect of the present disclosure provides a control method for controlling a surgical system having distension, shaving, and suction functions, comprising:
setting a predetermined value of a distension pressure for a target cavity;
activating a distension device of the surgical system, generating an activation signal for the distension device, recording an activation time of the distension device, and monitoring a pressure of distension fluid delivered to the target cavity in real time to monitor a pressure in the target cavity;
activating a negative pressure supply device of a collection device of the surgical system based at least on the activation signal for the distension device, suctioning and recovering tissue waste and fluid waste generated during a shaving operation, and generating an activation signal of the negative pressure supply device; and
activating a shaving device of the surgical system based at least on the activation signal of the negative pressure supply device, performing the shaving operation in the target cavity, and monitoring a lacking volume of the distension fluid in the target cavity in real time based on a total amount of the distension fluid delivered to the target cavity by the distension device and a total amount of the distension fluid recovered by the collection device,
wherein the negative pressure supply device and the shaving device are not activated until the pressure in the target cavity reaches the predetermined value.

The method of at least one embodiment of the present disclosure further comprises: based on the activation time of the distension device, determining whether the operating duration of the distension device is greater than or equal to a first duration threshold. If yes, generating a corresponding warning signal; based on the activation time of the distension device, determining whether an operating duration of the distension device is greater than or equal to a second duration threshold. If yes, generating a corresponding warning signal.

The method of at least one embodiment of the present disclosure further comprises: determining whether the pressure of the distension fluid delivered to the target cavity by the distension device is within a preset pressure range. If not, generating the corresponding warning signal; determining whether the pressure of the distension fluid delivered to the target cavity by the distension device is greater than or equal to the preset pressure maximum value.

The method of at least one embodiment of the present disclosure further comprises: if the pressure of the distension fluid delivered to the target cavity by the distension device is not within the preset pressure range, controlling the pumping rate of the pumping device of the distension device to make the pressure of the distension fluid within the preset pressure range.

The method of at least one embodiment of the present disclosure further comprises: if the pressure of the distension fluid delivered to the target cavity by the distension device is less than or greater than the predetermined pressure for a preset duration, stopping the shaving device and the negative pressure supply device.

The method of at least one embodiment of the present disclosure further comprises: if the lacking volume of distension fluid in the target cavity is greater than or equal to a maximum lacking volume, stopping the shaving device and the negative pressure supply device.

The method of at least one embodiment of the present disclosure further comprises: based on the activation signal of the distension device, generating a fan activation signal to make a fan of the distension device to cool the driving device of the distension device.

The method of at least one embodiment of the present disclosure further comprises: when the temperature of the driving device of the distension device is less than or equal to a first temperature threshold, the controller closes the fan of the distension device

The method of at least one embodiment of the present disclosure further comprises: when the temperature of the driving device of the distension device is greater than or equal to a second temperature threshold, the fan of the distension device is restarted by the controller.

The method of at least one embodiment of the present disclosure further comprises: determining whether the lacking volume of distension fluid is greater than or equal to a first lacking volume threshold, if yes, generating a corresponding warning signal; and determining whether the lacking volume of distension fluid is greater than or equal to a second lacking volume threshold, if yes, generating a corresponding warning signal.

The method of at least one embodiment of the present disclosure further comprises: monitoring an operating status of the pumping device and the driving device of the distension device, and when the operating status of the pumping device and/or the driving device of the distension device is in an abnormal state, the controller controls the shaving device, the negative pressure supply device of the collection device, and the distension device to shut down sequentially.

In the method of at least one embodiment of the present disclosure, the abnormal state includes at least one of overheating of the driving device, overcurrent of the operating current of the driving device, and stalling of the pumping device.

In the method of at least one embodiment of the present disclosure, the surgical system is the surgical system as described in any embodiment described above.

### DESCRIPTION OF DRAWINGS

The accompanying drawings illustrate exemplary embodiments of the present disclosure and, together with the description thereof, serve to explain the principles of the present disclosure, wherein these drawings are included to provide a further understanding of the present disclosure, and the drawings are incorporated in and constitute a part of this specification.
Fig. 1 is a schematic diagram of an overall structure of the surgical system having distension, shaving, and suction functions of one embodiment of the present disclosure.
Fig. 2 is a schematic diagram of overall structure of surgical system of one embodiment of the present disclosure.
Fig. 3 is a structural schematic diagram of the pressure measurement device of one embodiment of the present disclosure.
Fig. 4 is an overall structural schematic view of the shaving device 300 of one embodiment of the present disclosure.
Fig. 5 is a schematic view of an outer cutter and an inner cutter of the shaving device of one embodiment of the present disclosure.
Fig. 6 shows a partial structure of the outer cutter of the shaving device of one embodiment of the present disclosure.
Fig. 7 shows a partial structure of the inner cutter of the shaving device of one embodiment of the present disclosure.
Fig. 8 shows a flowchart of a control method of one embodiment of the present disclosure.

### DETAILED DESCRIPTION

The following provides a further detailed description of the present disclosure in conjunction with the accompanying drawings and embodiments. It is to be understood that the specific embodiments described herein are only used to explain the relevant content and are not intended to limit the present disclosure. It should also be noted that, for ease of description, the drawings only show portions related to the present disclosure.

It should be noted that, in the absence of conflict, the embodiments and features in the embodiments of the present disclosure can be combined with each other. The technical solutions of the present disclosure will be described in detail below with reference to the drawings and in conjunction with the embodiments.

Unless otherwise stated, the exemplary embodiments/examples shown are to be understood as providing exemplary features of various details of some ways in which the technical concepts of the present disclosure can be implemented in practice. Therefore, unless otherwise stated, the features of various embodiments/examples can be additionally combined, separated, interchanged, and/or rearranged without departing from the technical concepts of the present disclosure.

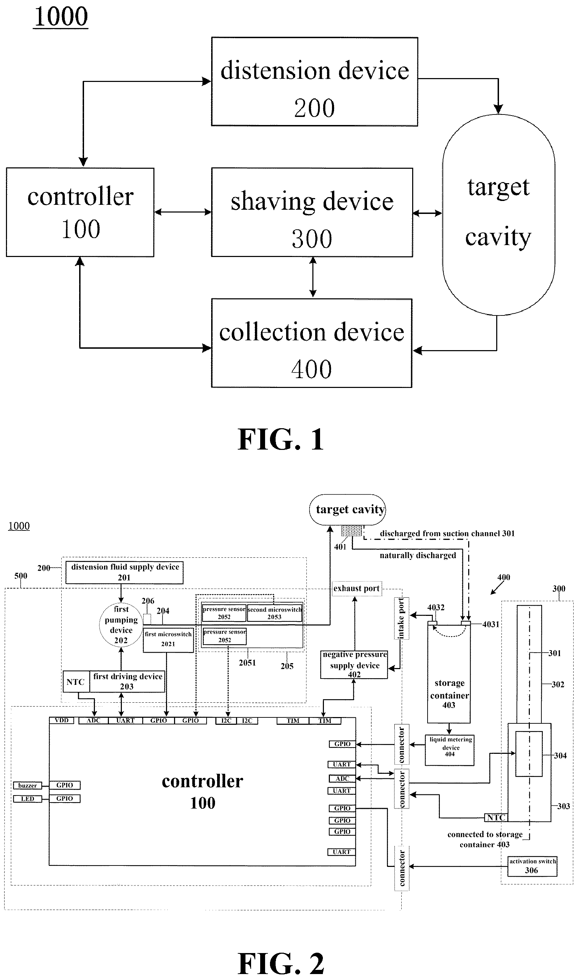

The following, in conjunction with Figs. 1 to 8, provides a detailed description of the surgical system having distension, shaving, and suction functions, the control method and the surgical apparatus of the present disclosure.

Fig. 1 is a schematic diagram of an overall structure of the surgical system having distension, shaving, and suction functions of one embodiment of the present disclosure.

Referring to Fig. 1, in some embodiments of the present disclosure, a surgical system 1000 having distension, shaving, and suction functions includes a controller 100, a distension device 200, a shaving device 300, and a collection device 400.

The distension device 200, based on control signals of the controller 100, delivers distension fluid to a target cavity to perform a distension operation in the target cavity. The shaving device 300, based on the control signals of the controller 100, performs a shaving operation in the target cavity. The shaving device 300 includes a suction channel 301.

The collection device 400 preferably includes a distension fluid collection device 401, a negative pressure supply device 402 and a storage container 403.

The distension fluid collection device 401 collects the distension fluid naturally discharged from the target cavity. The negative pressure supply device 402, based on the control signals of the controller 100, supply a negative pressure to the suction channel 301, such that the suction channel 301, based on the negative pressure, suctions tissue waste and fluid waste generated during the shaving operation. The storage container 403 is in fluid communication with both the suction channel 301 and the distension fluid collection device 401 to contain the tissue waste and the fluid waste from the suction channel 301 and the distension fluid from the distension fluid collection device 401.

The surgical system 1000 of the present disclosure may perform surgical operations on the human uterine cavity, bladder cavity, and the like.

The present disclosure, by configuring the distension device 200, the shaving device 300 and the collection device 400 in the surgical system 1000, and controlling the distension device 200, the shaving device 300, and the collection device 400 based on one controller 100, enhances an automation degree of the surgical system 1000 having distension, shaving, and suction functions of the present disclosure.

The controller 100 of the present disclosure may be a single-chip microcomputer, a microcontroller (MCU), or any processor having computer program processing functions. The present disclosure does not particularly limit the specific type or model of the controller 100.

Fig. 2 is a schematic diagram of overall structure of surgical system of one embodiment of the present disclosure.

Referring to Fig. 2, in some embodiments of the present disclosure, the distension device 200 of the surgical system 1000 includes a distension fluid supply device 201, a first pumping device 202 and a first driving device 203.

The distension fluid supply device 201 stores the distension fluid. The first driving device 203, based on the control signals of the controller 100, drives the first pumping device 202 such that the first pumping device 202 delivers the distension fluid supplied by the distension fluid supply device 201 to the target cavity via a delivery pipe 204.

The distension fluid supply device 201 may be in the form of an infusion liquid bag. Those skilled in the art can use a suitable liquid supply bag, bottle or the like as the distension fluid supply device 201 of the present disclosure. The distension fluid may be a commonly used medical distension fluid.

The first pumping device 202 of the present disclosure may be a commonly used infusion pump, for example, a peristaltic pump.

The first driving device 203 of the present disclosure is preferably a stepper motor, for example, an integrated stepper motor.

Fig. 2 exemplarily shows some communication interfaces of the controller 100, which should not be understood as a limitation for the controller 100. Those skilled in the art, under the inspiration of the present disclosure, can select the controller 100 having a corresponding interface number or model.

Referring to Fig. 2, in some embodiments of the present disclosure, the distension device 200 of the surgical system 1000 further includes a pressure measurement device 205.

The pressure measurement device 205 is used to measure a liquid pressure in the delivery pipe 204 to monitor a liquid pressure in the target cavity (for example, the human uterine cavity).

Since the liquid pressure in the target cavity is the same as the liquid pressure in the delivery pipe 204 (using a peristaltic pump), the distension device 200 of the present disclosure monitors the liquid pressure in the target cavity by measuring the pressure in the delivery pipe 204.

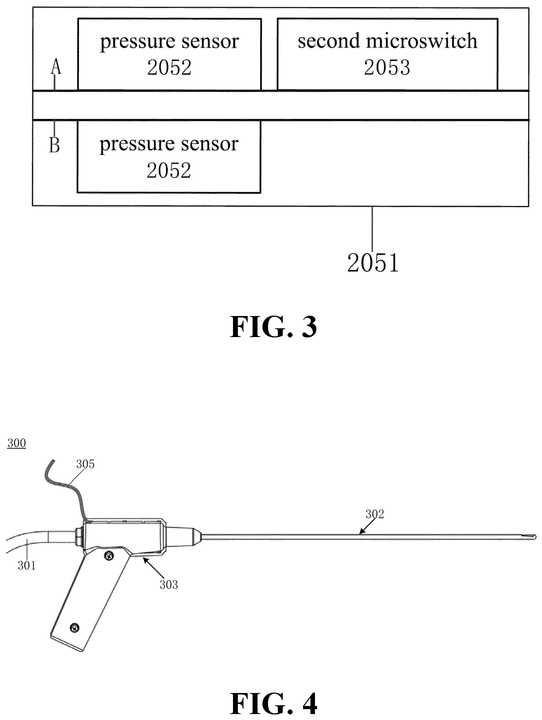

Fig. 3 is a structural schematic diagram of the pressure measurement device of one embodiment of the present disclosure.

In some embodiments of the present disclosure, referring to Fig. 3, the pressure measurement device 205 of the surgical system 1000 includes a pressure chamber 2051 and at least one pressure sensor 2052.

The pressure chamber 2051 has a first wall A and a second wall B. At least a portion of the delivery pipe 204 may be configured in a channel between the first wall A and the second wall B. The at least one pressure sensor 2052, for example, includes a first pressure sensor and a second pressure sensor. The pressure sensor 2052 is arranged on the first wall A, the second wall B or both the first wall A and the second wall B to measure the liquid pressure in the delivery pipe 204.

The pressure chamber 2051 may be a cylinder or other shape having a hollow channel. The delivery pipe 204 may pass through the hollow channel. The wall of the hollow channel serves as the first wall A and the second wall B described above. When the distension fluid is delivered in the delivery pipe 204, an outer wall of the delivery pipe 204 will contact the at least one pressure sensor 2052 arranged on the wall of the hollow channel of the pressure chamber 2051 to measure the liquid pressure in the delivery pipe 204.

The cross-section of the hollow channel of the pressure chamber 2051 is preferably circular, but may also be elliptical or rectangular.

The hollow channel of the pressure chamber 2051 may be a hollow channel that is circumferentially closed or a hollow channel that is circumferentially open.

The first wall A and the second wall B described above may be two regions of the wall of the hollow channel having a circular cross-section, preferably two regions opposite to each other.

Those skilled in the art should understand that the specific form of the pressure chamber may be adjusted, all of which fall within the protection scope of the present disclosure.

The delivery pipe 204 described above is a flexible pipe, for example, a rubber tube.

In some embodiments of the present disclosure, the pressure sensor 2052 of the surgical system 1000 is configured in an embedded manner in the first wall A, the second wall B or both the first wall A and the second wall B.

The liquid pressure measured by the pressure measurement device 205 of the surgical system 1000 may be transmitted to the controller 100 in real time.

In some embodiments of the present disclosure, referring to Fig. 2, the first pumping device 202 of the surgical system 1000 is equipped with a first microswitch 2021. When the delivery pipe 204 is configured ready with the first pumping device 202, the first microswitch 2021 generates a first trigger signal, and the first trigger signal is transmitted to the controller 100.

In some embodiments of the present disclosure, referring to Figs. 2 and 3, the pressure chamber 2051 of the surgical system 1000 is preferably equipped with a second microswitch 2053. When the delivery pipe 204 is arranged ready between the first wall A and the second wall B, the second microswitch 2053 generates a second trigger signal, and the second trigger signal is transmitted to the controller 100.

The distension device 200, by being equipped with the first microswitch 2021 and the second microswitch 2053, may detect the configuration status of the delivery pipe 204 in real time, to determine whether the delivery pipe 204 has been configured ready with the first pumping device 202 and the pressure chamber 2051.

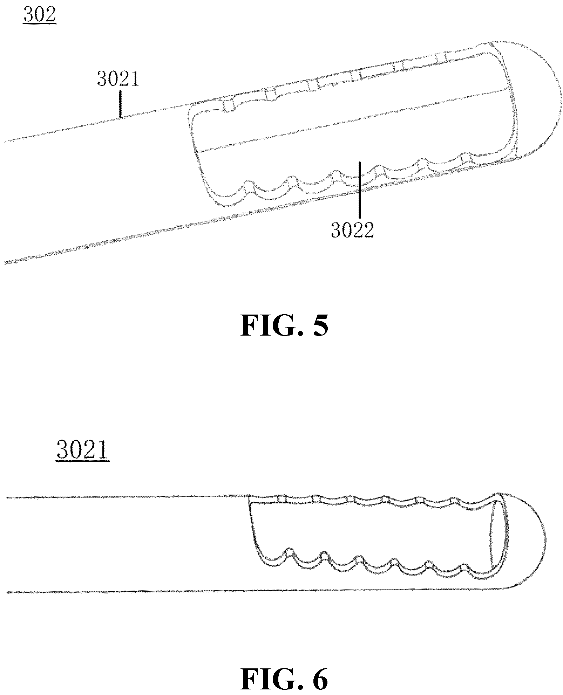

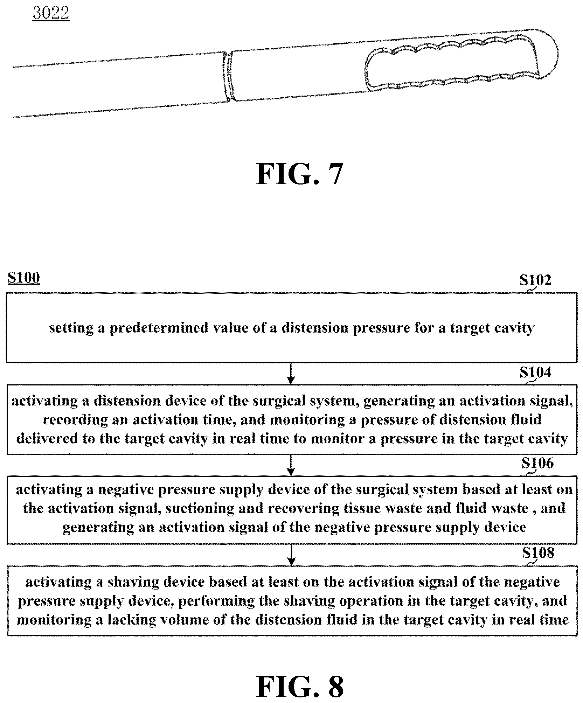

Fig. 4 is an overall structural schematic view of the shaving device 300 of one embodiment of the present disclosure. Fig. 5 is a schematic view of an outer cutter and an inner cutter of the shaving device of one embodiment of the present disclosure. Fig. 6 shows a partial structure of the outer cutter of the shaving device of one embodiment of the present disclosure. Fig. 7 shows a partial structure of the inner cutter of the shaving device of one embodiment of the present disclosure.

Referring to Figs. 4 to 7, in some embodiments of the present disclosure, the shaving device 300 of the surgical system 1000 includes shaving cutters 302 and a shaving operation device 303.

The shaving cutters 302 include the outer cutter 3021 and the inner cutter 3022. The inner cutter 3022 is arranged within the outer cutter 3021 and coaxially aligned with the outer cutter 3021.

The shaving operation device 303 drives the inner cutter 3022 of the shaving cutters 302, such that the inner cutter 3022 may be rotated relative to the outer cutter 3021 to perform the shaving operation described above.

The suction channel 301 is formed within the inner cutter 3022 and within the shaving operation device 303 to communicate the target cavity and the negative pressure supply device 402.

Preferably, in some embodiments of the present disclosure, both the inner cutter and the outer cutter of the shaving cutters 302 are tubular.

Referring to Figs. 6 and 7, in some embodiments of the present disclosure, a first end of the inner cutter 3022 has a side opening served as a blade of the inner cutter. A second end of the inner cutter 3022 is driven by the shaving operation device 303. A first end of the outer cutter 3021 has a side opening served as a blade of the outer cutter. A second end of the outer cutter 3021 is fixedly connected to the shaving operation device 303.

The side opening of the first end of the inner cutter 3022 and the side opening of the first end of the outer cutter 3021 have a same or similar axial position.

Fig. 4 also shows a cable 305. The cable 305 is used to receive motor drive signals from the controller 100 and transmit the motor drive signals to a motor 304 arranged within the shaving operation device 303.

Referring to Fig. 2, the suction channel 301 is in communication with a first port 4031 of the storage container 403. The negative pressure supply device 402 is in communication with a second port 4032 of the storage container 403. The negative pressure supply device 402, via a gas passage between the first port 4031 and the second port 4032 (see the dashed arc with an arrow), provides the negative pressure to the suction channel 301 to suction the tissue waste and the fluid waste generated during the shaving operation.

In some embodiments of the present disclosure, the distension fluid collection device 401 is also in communication with the first port 4031 of the storage container 403. The negative pressure supply device 402, via the gas passage between the first port 4031 and the second port portion 4032, also provides the negative pressure to the distension fluid collection device 401 to collect the distension fluid naturally discharged from the target cavity.

The distension fluid collection device 401 may be in the form of a collection bag.

In some embodiments of the present disclosure, the collection device 400 of the surgical system 1000 further includes a liquid metering device 404.

The liquid metering device 404 measures a liquid volume in the storage container 403 in real time and transmits the measurement result to the controller 100 in real time. The liquid metering device 404 may be a force sensor.

In some embodiments of the present disclosure, referring to Fig. 2, the shaving device 300 of the surgical system 1000 further includes an activation switch 306.

The activation switch 306, based on an operation action, generates an activation signal for the shaving device and transmits the activation signal to the controller 100. The controller 100, at least based on the activation signal of the shaving device, generates the motor drive signals to drive the motor 304 of the shaving operation device 303.

The activation switch 306 is preferably a foot-operated switch.

In some embodiments of the present disclosure, the distension device 200 further includes a flow detection device 206. The flow detection device 206 measures a volume of the distension fluid delivered to the target cavity by the distension device 200 in real time and transmits the measurement value to the controller 100 in real time.

The flow detection device 206 may be a flow meter and may be arranged at a connection place of the first pumping device 202 and the delivery pipe 204.

In some preferred embodiments of the present disclosure, the controller 100, at least based on a first trigger signal of a first microswitch 2021 and a second trigger signal of a second microswitch 2053, generates the control signals for controlling the first driving device 203.

After the delivery pipe 204 is configured ready with the first pumping device 202 and the delivery pipe is configured ready with the pressure chamber 2051, the controller 100 may control the first driving device 203 to drive the first pumping device 202, such that the first pumping device 202 performs a delivery of the distension fluid.

Referring to Fig. 2, some components of the surgical system 1000 can be configured in the form of an integrated machine as a surgical apparatus 500. The components are shown in the largest dashed box in Fig. 2.

The surgical apparatus 500 may cooperate with the shaving device 300, the collection device 400 and the distension fluid supply device 201.

Referring to Fig. 2, the surgical apparatus 500 may be configured with an air intake port and an exhaust port to enable the negative pressure supply device 402 to operate.

The surgical apparatus 500 may also be configured with multiple connectors to enable the controller 100 to send and receive signals via the connectors.

In some embodiments of the present disclosure, the surgical apparatus 500 is also configured with a thermistor of negative temperature coefficient (NTC) for monitoring an operating temperature of the first driving device 203.

The shaving device 300 may also be configured with a thermistor of negative temperature coefficient (NTC) for monitoring an operating temperature (for example, battery temperature) of the shaving operation device 303.

It should be noted that the surgical system shown in Fig. 1 should not be understood as a limitation to the surgical system of the present disclosure. Fig. 1 is only used to illustrate the surgical system and surgical apparatus of the present disclosure.

In some embodiments of the present disclosure, the surgical system 1000 further includes a first operation receiving device. The first operation receiving device is used to receive user's operation actions (a touch operation or a press operation) to generate a first operation signal and transmit the first operation signal to the controller 100. The controller 100, based on the first trigger signal, the second trigger signal, and the first operation signal, generates the control signals for controlling the first driving device 203.

The first operation receiving device may be in the form of a button, for example, a button configured on the housing of the surgical apparatus 500, or in the form of a touchscreen, for example, a partial touch area of a touchscreen configured on the housing of the surgical apparatus 500.

The operation actions received by the first operation receiving device may include an activation operation of the first driving device 203. The first operation signal can be an activation control signal of the first driving device 203.

In some embodiments of the present disclosure, the activation of the first driving device 203 depends on the first trigger signal and the second trigger signal.

In some embodiments of the present disclosure, the surgical system 1000 further includes a second operation receiving device. The second operation receiving device is used to receive user's operation actions (a touch operation or a press operation) to generate a second operation signal and transmit the second operation signal to the controller 100. The controller 100, at least based on the second operation signal, generates the control signals for controlling the negative pressure supply device 402 to provide the negative pressure.

The second operation receiving device may be in the form of a button, for example, a button configured on the housing of the surgical apparatus 500, or in the form of a touchscreen, for example, a partial touch area of a touchscreen configured on the housing of the surgical apparatus 500.

Preferably, the controller 100 of the present disclosure, based on the second operation signal and the control signals generated by the controller 100 for controlling the first driving device 203, generates the control signals for controlling the negative pressure supply device 402 to provide the negative pressure.

The operation actions received by the second operation receiving device can include an activation operation of the negative pressure supply device 402. The second operation signal can be an activation control signal of the negative pressure supply device 402.

In some preferred embodiments of the present disclosure, the activation of the negative pressure supply device 402 depends on the activation of the first driving device 203.

In some embodiments of the present disclosure, the surgical system 1000 further includes a third operation receiving device. The third operation receiving device is used to receive user's operation actions (a touch operation or a press operation) to generate a third operation signal and transmit the third operation signal to the controller 100. The controller 100, based on the third operation signal, associates a preset operating mode (rotational speed, etc.) for the shaving cutters. The controller 100, based on the activation signal (generated by the activation switch 306) for the shaving device and the third operation signal, outputs a shaving control signal associated with the preset operating mode to control the shaving operation device 303 to drive the inner cutter.

The third operation receiving device may be in the form of a button, for example, a button configured on the housing of the surgical apparatus 500, or in the form of a touchscreen, for example, a partial touch area of a touchscreen configured on the housing of the surgical apparatus 500.

The operation actions received by the third operation receiving device may include an operation for selecting an operating mode for the shaving device.

It should be noted that the shaving cutters 302 of the shaving device 300 may enter the target cavity via an endoscope (not shown) arranged outside the surgical system 1000 to perform the shaving operation.

The delivery pipe 204 may also deliver the distension fluid to the target cavity via the endoscope.

Referring to Figs. 1 to 7, the present disclosure also provides a surgical apparatus 500 having distension, shaving, and suction functions. The surgical apparatus 500 may include the controller 100, the distension device 200, the shaving operation device, and the negative pressure supply device 402:
The distension device 200, based on the control signals of the controller 100, delivers the distension fluid to the target cavity to perform the distension operation in the target cavity.

The shaving operation device, based on the control signals of the controller 100, drives the shaving device having the suction channel 301 outside the surgical apparatus to perform the shaving operation in the target cavity.

The negative pressure supply device 402, based on the control signals of the controller 100, provides the negative pressure, such that the suction channel 301 of the shaving device 300 may suction the tissue waste and the fluid waste generated during the shaving operation based on the negative pressure.

In some embodiments of the present disclosure, the distension device 200 of the surgical apparatus 500 may include the first pumping device 202 and the first driving device 203.

The first driving device 203, based on the control signals of the controller 100, drives the first pumping device 202 such that the first pumping device 202 delivers the distension fluid provided by a distension fluid supply device 201 arranged outside the surgical apparatus to the target cavity via the delivery pipe 204.

In some embodiments of the present disclosure, the distension device 200 of the surgical apparatus 500 further includes the pressure measurement device 205.

The pressure measurement device 205 is used to measure the liquid pressure in the delivery pipe 204 to monitor the liquid pressure in the target cavity.

Preferably, the pressure measurement device 205 includes the pressure chamber 2051 and the at least one pressure sensor 2052.

The pressure chamber 2051 has the first wall and the second wall. The at least a portion of the delivery pipe 204 may be configured between the first wall and the second wall.

The at least one pressure sensor 2052, for example, includes the first pressure sensor and the second pressure sensor. The pressure sensor 2052 is configured on the first wall and/or the second wall to measure the liquid pressure in the delivery pipe 204.

In some embodiments of the present disclosure, the delivery pipe 204 of the surgical apparatus 500 is a flexible pipe.

In some embodiments of the present disclosure, the pressure sensor 2052 of the surgical apparatus 500 is configured in an embedded manner on the first wall and/or the second wall.

In some embodiments of the present disclosure, the liquid pressure measured by the pressure measurement device 205 of the surgical apparatus 500 is transmitted to the controller 100 in real time.

In some embodiments of the present disclosure, the first pumping device 202 of the surgical apparatus 500 is configured with the first microswitch 2021. When the delivery pipe 204 is configured ready with the first pumping device 202, the first microswitch 2021 generates the first trigger signal, and the first trigger signal is transmitted to the controller 100.

In some embodiments of the present disclosure, the first pumping device 202 of the surgical apparatus 500 is a peristaltic pump, and the first driving device 203 is a stepper motor.

In some embodiments of the present disclosure, the pressure chamber 2051 of the surgical apparatus 500 is configured with the second microswitch 2053. When the delivery pipe 204 is configured ready between the first wall and the second wall, the second microswitch 2053 generates the second trigger signal, and the second trigger signal is transmitted to the controller 100.

The present disclosure also provides a control method that may be used to control the surgical system 1000 of any embodiment of the present disclosure.

Referring to Fig. 8, in some embodiments of the present disclosure, the control method S100 may include steps S102 to S108.

The step S102 may include: setting a predetermined value of the distension pressure of the target cavity. The predetermined value of the distension pressure of the target cavity may be set based on a button operation or a touch operation. For example, it can be set based on a control panel or touchscreen of an integrated surgical apparatus described above.

The step S104 may include: activating the distension device 200 of the surgical system 1000, generating the activation signal of the distension device, recording the activation time of the distension device, and monitoring the pressure of the distension fluid delivered to the target cavity by the distension device 200 in real time to monitor the pressure in the target cavity. The distension device 200 of the surgical system 1000 can be activated by performing an operation action on the first operation receiving device of the control panel or touchscreen described above.

The step S106 may include: at least based on the activation signal of the distension device, activating the negative pressure supply device 402 of the collection device 400 of the surgical system 1000 to suction and recover the tissue waste and the fluid waste generated during the shaving operation, and generating the activation signal for the negative pressure supply device. The negative pressure supply device 402 may be activated by performing an operation action on the second operation receiving device of the control panel or touchscreen described above. In the control method of the present disclosure, the distension device 200 must be activated before the negative pressure supply device 402 is activated.

The step S108 may include: at least based on the activation signal of the negative pressure supply device, activating the shaving device 300 of the surgical system to perform the shaving operation in the target cavity, and monitoring a lacking volume of the distension fluid in the target cavity in real time based on a total amount of the distension fluid delivered to the target cavity by the distension device 200 and a total amount of the distension fluid suctioned and recovered by the collection device 400. In the control method of the present disclosure, the negative pressure supply device 402 must be activated before the shaving device 300 is activated.

Before the pressure in the target cavity reaches the predetermined value of the distension pressure, the negative pressure supply device and the shaving device cannot be activated.

In some embodiments of the present disclosure, the control method S100 may further include the following.

The method may include: based on the activation time of the distension device, determining whether the operating duration of the distension device is greater than or equal to a first duration threshold. If yes, generating a corresponding warning signal (for example, a low-frequency audio-visual warning signal).

The method may include: based on the activation time of the distension device, determining whether an operating duration of the distension device is greater than or equal to a second duration threshold. If yes, generating a corresponding warning signal (for example, a high-frequency audio-visual warning signal).

In some embodiments of the present disclosure, the integrated surgical apparatus may further include a warning device, for example, an audio-visual warning device.

In some embodiments of the present disclosure, the control method S100 may further include the following.

The method may include: determining whether the pressure of the distension fluid delivered to the target cavity by the distension device is within a preset pressure range. If not, generating the corresponding warning signal (for example, the low-frequency audio-visual warning signal).

The method may include: determining whether the pressure of the distension fluid delivered to the target cavity by the distension device is greater than or equal to the preset pressure maximum value (for example, 200 mmHg). If yes, generating the corresponding warning signal (for example, the high-frequency audio-visual warning signal).

In some embodiments of the present disclosure, the control method S100 may further include: if the pressure of the distension fluid delivered to the target cavity by the distension device is not within the preset pressure range, controlling the pumping rate of the pumping device of the distension device (i.e., the first pumping device described above) to make the pressure of the distension fluid within the preset pressure range.

In some embodiments of the present disclosure, the method may further include: if the pressure of the distension fluid delivered to the target cavity by the distension device is less than or greater than the predetermined pressure for a preset duration, stopping the shaving device and the negative pressure supply device.

In some embodiments of the present disclosure, the method may further include: if the lacking volume of distension fluid in the target cavity is greater than or equal to a maximum lacking volume, stopping the shaving device and the negative pressure supply device.

In some embodiments of the present disclosure, the control method S100 further includes: based on the activation signal of the distension device, generating a fan activation signal to make a fan of the distension device to cool the driving device of the distension device (i.e., the first driving device, which may be a stepper motor).

In some embodiments of the present disclosure, the integrated surgical apparatus may further include a fan device. The fan device is controlled by the controller 100 described above.

In some embodiments of the present disclosure, when the temperature of the driving device of the distension device 200 (i.e., the first driving device 203 described above) is less than or equal to a first temperature threshold (for example, 40°C), the controller 100 closes the fan of the distension device. Referring to Fig. 2, the temperature of the driving device of the distension device 200 may be measured by a thermistor of negative temperature coefficient (NTC).

In some embodiments of the present disclosure, when the temperature of the driving device of the distension device 200 is greater than or equal to a second temperature threshold (for example, 70°C), the fan of the distension device is restarted by the controller 100.

In some embodiments of the present disclosure, the control method S100 further includes the following.

The method may include: determining whether the lacking volume of distension fluid is greater than or equal to a first lacking volume threshold (for example, 750 mL). If yes, generating a corresponding warning signal (for example, a low-frequency audio-visual warning signal).

The method may include: determining whether the lacking volume of distension fluid is greater than or equal to a second lacking volume threshold (for example, 2000 mL). If yes, generating a corresponding warning signal (for example, a high-frequency audio-visual warning signal).

In some embodiments of the present disclosure, the control method S100 further includes: monitoring an operating status of the pumping device and the driving device of the distension device 200. When the operating status of the pumping device and/or the driving device of the distension device 200 is in an abnormal state, the controller 100 controls the shaving device 300, the negative pressure supply device 402 of the collection device, and the distension device 200 to shut down sequentially.

The abnormal state includes but is not limited to at least one of overheating of the driving device, overcurrent of the operating current of the driving device, or stalling of the pumping device.

In the description of this specification, references to terms such as "one embodiment/manner" "some embodiments/manners" "example" "specific example" or "some examples" mean that the specific features, structures, materials, or characteristics described in connection with the embodiment/manner or example are included in at least one embodiment/manner or example of the present disclosure. In this specification, the schematic expressions of the above terms do not necessarily refer to the same embodiment/manner or example. Moreover, the specific features, structures, materials, or characteristics described may be combined in any suitable manner in any one or more embodiments/manners or examples. Furthermore, in the absence of contradiction, those skilled in the art may combine and integrate different embodiments/manners or examples described in this specification, as well as features of different embodiments/manners or examples.

In addition, the terms "first" and "second" are used for descriptive purposes only and cannot be understood as indicating or implying relative importance or implicitly specifying the number of technical features indicated. Thus, features defined with "first" or "second" may explicitly or implicitly include at least one such feature. **In** the description of the present disclosure, "multiple" means at least two, for example, two, three, etc., unless explicitly and specifically limited otherwise.

Those skilled in the art should understand that the above embodiments are merely for clearly illustrating the present disclosure and are not intended to limit the scope of the present disclosure. For those skilled in the art, other changes or variations may be made based on the above disclosure, and such changes or variations still fall within the scope of the present disclosure.

## Claims

1. A surgical system having distension, shaving and suction functions, **characterized in that**, comprising:
a controller;
a distension device configured to deliver distension fluid to a target cavity and perform a distension operation based on control signals of the controller;
a shaving device configured to perform a shaving operation in the target cavity based on the control signals of the controller, wherein the shaving device has a suction channel; and
a collection device,
wherein the collection device comprises:
a distension fluid collection device configured to collect the distension fluid naturally discharged from the target cavity;
a negative pressure supply device configured to supply a negative pressure to the suction channel based on the control signals of the controller, such that the suction channel suctions tissue waste and fluid waste generated during the shaving operation; and
a storage container configured to be in fluid communication with both the suction channel and the distension fluid collection device, and contain the tissue waste and the fluid waste from the suction channel and the distension fluid from the distension fluid collection device.

2. The surgical system of claim 1, **characterized in that** the distension device comprises:
a distension fluid supply device configured to store the distension fluid;
a first pumping device; and
a first driving device configured to drive the first pumping device based on the control signals of the controller, such that the first pumping device delivers the distension fluid from the distension fluid supply device to the target cavity via a delivery pipe.

3. The surgical system of claim 2, **characterized in that** the distension device further comprises a pressure measurement device that measures a liquid pressure in the delivery pipe to monitor a liquid pressure in the target cavity.

4. The surgical system of claim 3, **characterized in that** the pressure measurement device comprises:
a pressure chamber having a first wall and a second wall, wherein at least a portion of the delivery pipe is arranged between the first wall and the second wall; and
at least one pressure sensor arranged on the first wall, the second wall or both the first wall and the second wall to measure the liquid pressure in the delivery pipe.

5. The surgical system of claim 2, **characterized in that** the first pumping device is equipped with a first microswitch, wherein the first microswitch generates a first trigger signal when the delivery pipe is connected with the first pumping device, and the first trigger signal is transmitted to the controller.

6. The surgical system of claim 5, **characterized in that** the pressure chamber is equipped with a second microswitch, wherein the second microswitch generates a second trigger signal when the delivery pipe is properly arranged between the first wall and the second wall, and the second trigger signal is transmitted to the controller.

7. The surgical system of claim 1, **characterized in that** the shaving device comprises:
shaving cutters having an outer cutter and an inner cutter, wherein the inner cutter is disposed within the outer cutter and coaxially aligned with the outer cutter; and
a shaving operation device configured to drive the inner cutter to rotate relative to the outer cutter to perform the shaving operation,
wherein the suction channel is formed within the inner cutter and the shaving operation device, and communicates the target cavity and the negative pressure supply device.

8. The surgical system of claim 7, **characterized in that** the inner cutter and the outer cutter are tubular, the inner cutter has a first end having a side opening serving as a blade of the inner cutter, the inner cutter has a second end being driven by the shaving operation device, the outer cutter has a first end having a side opening serving as a blade of the outer cutter, and the outer cutter has a second end being fixedly connected to the shaving operation device, wherein the side opening of the first end of the inner cutter and the side opening of the first end of the outer cutter have a same or similar axial position.

9. The surgical system of claim 7, **characterized in that** the storage container has a first port being in communication with the suction channel, the storage container has a second port being in communication with the negative pressure supply device, and the negative pressure supply device supplies the negative pressure to the suction channel via a gas passage arranged between the first port and the second port to suction the tissue waste and the fluid waste.

10. The surgical system of claim 9, **characterized in that** the distension fluid collection device is in communication with the first port of the storage container, and the negative pressure supply device supplies the negative pressure to the distension fluid collection device via the gas passage to collect the distension fluid naturally discharged from the target cavity.

11. The surgical system of claim 1, **characterized in that** the collection device further comprises a liquid metering device, and the liquid metering device measures a liquid volume in the storage container in real time and transmits the liquid volume to the controller in real time.

12. The surgical system of claim 6, **characterized in that** the controller generates the control signals to control the first driving device, based at least on the first trigger signal of the first microswitch and the second trigger signal of the second microswitch.

13. The surgical system of claim 12, **characterized in that** the surgical system further comprises a first operation receiving device that receives user's operation actions to generate a first operation signal and transmits the first operation signal to the controller, and the controller generates the control signals to control the first driving device based on the first trigger signal, the second trigger signal and the first operation signal.

14. The surgical system of claim 12, **characterized in that** the surgical system further comprises a second operation receiving device that receives user's operation actions to generate a second operation signal and transmits the second operation signal to the controller, and the controller generates the control signals to control the negative pressure supply device to supply the negative pressure based at least on the second operation signal.

15. The surgical system of claim 7, **characterized in that** the surgical system further comprises a third operation receiving device that receives user's operation actions to generate a third operation signal and transmits the third operation signal to the controller, the controller associates a preset operating mode for the shaving cutters based on the third operation signal, and the controller outputs a shaving control signal associated with the preset operating mode to control the shaving operation device to drive the inner cutter based on the third operation signal and an activation signal for the shaving device.

16. The surgical system of claim 7, **characterized in that** the shaving cutters enter the target cavity via an endoscope located outside the surgical system, and perform the shaving operation.

17. A surgical apparatus having distension, shaving, and suction functions, **characterized in that** comprising:
a controller;
a distension device configured to deliver distension fluid to a target cavity to perform a distension operation based on control signals of the controller;
a shaving operation device configured to drive a shaving device and perform a shaving operation in the target cavity based on the control signals of the controller, wherein the shaving device locates outside the surgical apparatus and has a suction channel; and
a negative pressure supply device configured to supply a negative pressure based on the control signals of the controller, such that the suction channel of the shaving device suctions tissue waste and fluid waste generated during the shaving operation.

18. A control method for controlling a surgical system having distension, shaving, and suction functions, **characterized in that** comprising:
setting a predetermined value of a distension pressure for a target cavity;
activating a distension device of the surgical system, generating an activation signal for the distension device, recording an activation time of the distension device, and monitoring a pressure of distension fluid delivered to the target cavity in real time to monitor a pressure in the target cavity;
activating a negative pressure supply device of a collection device of the surgical system based at least on the activation signal for the distension device, suctioning and recovering tissue waste and fluid waste generated during a shaving operation, and generating an activation signal of the negative pressure supply device; and
activating a shaving device of the surgical system based at least on the activation signal of the negative pressure supply device, performing the shaving operation in the target cavity, and monitoring a lacking volume of the distension fluid in the target cavity in real time based on a total amount of the distension fluid delivered to the target cavity by the distension device and a total amount of the distension fluid recovered by the collection device,
wherein the negative pressure supply device and the shaving device are not activated until the pressure in the target cavity reaches the predetermined value.
